# EUROPEAN PATENT APPLICATION

(11) **EP 4 806 454 A1**
(43) Date of publication of application: **16.09.2026**
(21) Application number: 25163260.0
(22) Date of filing: 12.03.2025
(51) Int. Cl.: A61K 38/18, A61P 27/06

(54) **NGF FOR THE USE IN IMPROVING VISUAL FUNCTION IN GLAUCOMATOUS PATIENTS**

(71) Applicant: Dompé farmaceutici SpA, 20122 Milano (IT)
(72) Inventor: VECCHIOTTI, Davide, 67100 L'Aquila (IT); ZAZZERONI, Francesca, 67100 L'Aquila (IT); ALESSE, Edoardo, 67100 L'Aquila (IT); CIMINI, Annamaria, 67019 L'Aquila (IT); TESSITORE, Alessandra, 67100 L'Aquila (IT); CYPRIANO DUTRA, Rafael, 67100 L'Aquila (IT); CATTANI, Franca, 67100 L'Aquila (IT); ALLEGRETTI, Marcello, 67100 L'Aquila (IT); ARAMINI, Andrea, 67100 L'Aquila (IT)
(74) Representative: Dompé farmaceutici Spa

(57) **Abstract**

The present invention relates to nerve growth factor (NGF) for use in improving visual function in a subject suffering from glaucoma, wherein said NGF is administered intravitreally to said subject.

## Description

### FIELD OF THE INVENTION

The present invention relates to a method for improving visual function in glaucomatous patients.

### STATE OF THE ART

Glaucoma, one of the leading causes of blindness throughout the world, encompasses a group of ocular degenerative disorders characterized by progressive retinal ganglion cells degeneration and optic neuropathy.

A number of mechanisms have been invoked to explain RGC degeneration in glaucoma, including chronic intermittent ischemia, oxidative stress, inflammation, excitotoxicity, defective axon transport, trophic factor withdrawal, and loss of electrical activity (Chang EE et al, Ophthalmology 2012 May, 119(5): 979-86).

In most cases, glaucoma is associated with an inadequate drainage of aqueous humor through outflow pathways. Poor drainage results in the accumulation of aqueous humor and may cause IOP elevation, which contributes to irreversible, progressive damage to retinal ganglion cells (RGC) and resultant retinopathy and optic neuropathy.

Based on the anterior chamber anatomy and mechanism of aqueous humor outflow impairment, glaucoma is mainly classified in two main categories:
▪ open-angle glaucoma, with optic nerve damage associated with an open irido-corneal drainage angle. This is usually associated with an increased resistance to aqueous outflow through the trabecular meshwork. Intraocular pressure (IOP) is usually elevated but in some cases may be within the average range (normal-tension glaucoma). This type of glaucoma is typically asymptomatic in early stages and progresses slowly.
▪ angle-closure glaucoma, with optic nerve damage associated with a physical obstruction of the irido-corneal drainage angle. This type of glaucoma can be acute, where a sudden and severe obstruction and increase in IOP causes pain and vision loss, or chronic, with a gradual angle narrowing and increase in IOP leading to a progressive visual function degeneration.

These two types of glaucoma can then be further classified based on their underlying cause into primary, when no cause of outflow blockage is identified or secondary, when the outflow obstruction results from an identifiable cause, such as trauma, inflammation, neovascularization, or medication use.

In a patient with a diagnosis of glaucoma, progression and severity of the pathology may be evaluated on the basis on the abnormalities in the visual field, measured via the Humphrey 24-2 visual field (VF) test.

According to this classification system, the following four stages of glaucoma can be identified:
- Early stage (also known as mild glaucoma), with a mean deviation in the Humphrey 24-2 visual field (VF) test better than -6dB. This stage is characterized by minimal or not detectable vision loss.
- Intermediate stage (also known as moderate glaucoma) with a mean deviation in the Humphrey 24-2 visual field (VF) test between -6.01 and -12 dB. This stage is characterized by mild to moderate visual impairment, with preservation of central vision.
- Advanced stage (also known as severe glaucoma), with a mean deviation in the Humphrey 24-2 visual field (VF) test between -12.01 and -20 dB. This stage is characterized by noticeable visual impairment, including difficulty with contrast sensitivity and night vision.
- Late stage (also known as end stage glaucoma) with a mean deviation in the Humphrey 24-2 visual field (VF) test worse than -20.01 dB. This stage is characterized by severe vision loss, tunnel vision and legal blindness.

Currently, the only existing treatment for glaucoma consists in reducing the intraocular pressure (IOP). This is achieved by topical pharmacological treatments effective in lowering intraocular pressure via different mechanisms of action, or by the more invasive laser or incisional surgeries to improve aqueous outflow or establish a new outflow pathway for the aqueous humor (Weiner et al, JAMA 2014 14; 311(18): 1901-1911, Bedrood S. et al., Clin Ophthalmol. 2023 14;17: 3899-3913).

The above approach cannot reverse the damage to the optic nerve and recover vision loss but only aim to block or slow progression of the disease and prevent further optic nerve and visual field damage. Furthermore, in many patients glaucoma progression is slowed but still continues even after a significant IOP reduction has been achieved.

Therefore, there is a great unmet need for the development of new therapeutic approaches complementary to IOP reduction, effective in reversing dysfunction the damaged RGCs and attaining visual function improvement in glaucoma patients.

The development of new therapies for glaucoma is particularly challenging also due to the fact that some administration routes are generally not indicated for treating glaucoma, in order to avoid the risk of worsening visual loss, and/or accelerating the degenerative process of this disease; for example, caution is advised with the use of the intravitreal administration route as this may cause acute and chronic postinjection IOP increase, which may promote glaucomatous damage in susceptible optic nerves (Levin AM., et al., J Glaucoma. 2021 Dec 1;30(12):1019-1026).

In the light of the above, it is strongly felt the need to develop new effective and safe therapeutic approaches to glaucoma, possibly to improve the visual function of glaucomatous subjects.

### SUMMARY OF THE INVENTION

As it will described in the experimental section, the present inventors have now demonstrated that the intravitreal administration of nerve growth factor (NGF) unexpectedly restores visual function in a genetic model of glaucoma.

In particular, NGF administered intravitreally significantly restores the function of RGCs, in particular in intermediate and advanced stages of the pathology, when these cells are damaged, but not yet dead, in glaucomatous eyes.

Surprisingly, as demonstrated in the Experimental section, the intravitreal administration of NGF does not induce an increase in the intraocular pressure (IOP), in the subjects receiving said intravitreal administration.

The data obtained support the intravitreal administration of NGF to improve visual function in patients having at least a partial vision loss caused by glaucoma, with a more relevant effect in intermediate and advanced stage of the glaucomatous pathology.

Accordingly, a first object of the invention is nerve growth factor (NGF) for use in improving visual function in a subject suffering from glaucoma, said NGF being administered intravitreally to said subject.

A further object of the invention is a pharmaceutical composition comprising nerve growth factor (NGF), and at least one pharmaceutically acceptable excipient, for use in improving visual function in a subject suffering from glaucoma, said pharmaceutical composition being administered intravitreally to said subject.

### BRIEF DESCRIPTION OF THE FIGURES

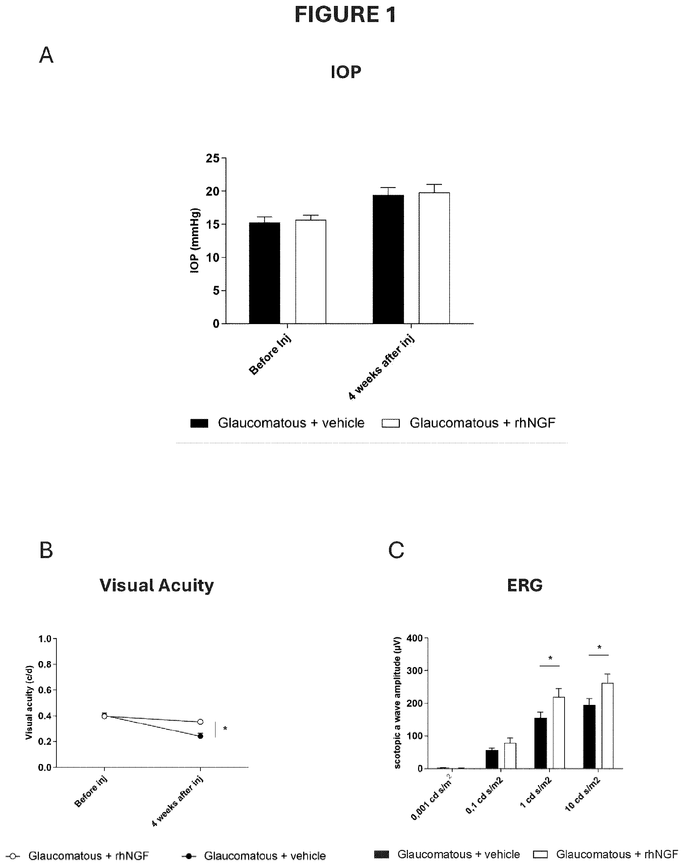

**Figure 1** shows the results of (A) intraocular pressure ("IOP", mmHg) and (B) visual acuity (c/d) analysis, before rhNGF or vehicle intravitreal injection ("Before inj."), and 4 weeks after rhNGF or vehicle intravitreal injection ("4 weeks after inj.") in 4-month-old animal groups, and the results of (C) scotopic a wave amplitude (µV) of ERG (at 0.001 cd·s/m², 0.1 cd·s/m², 1 cd·s/m² and 10 cd·s/m²) analysis of vehicle-treated glaucoma ("Glaucomatous + vehicle") and rhNGF-treated glaucoma ("Glaucomatous + rhNGF") 4-month-old animal groups, according to Example 1. Two-tailed Student's t-test. *p<0.05 vs. vehicle-treated glaucoma ("Glaucomatous + vehicle").

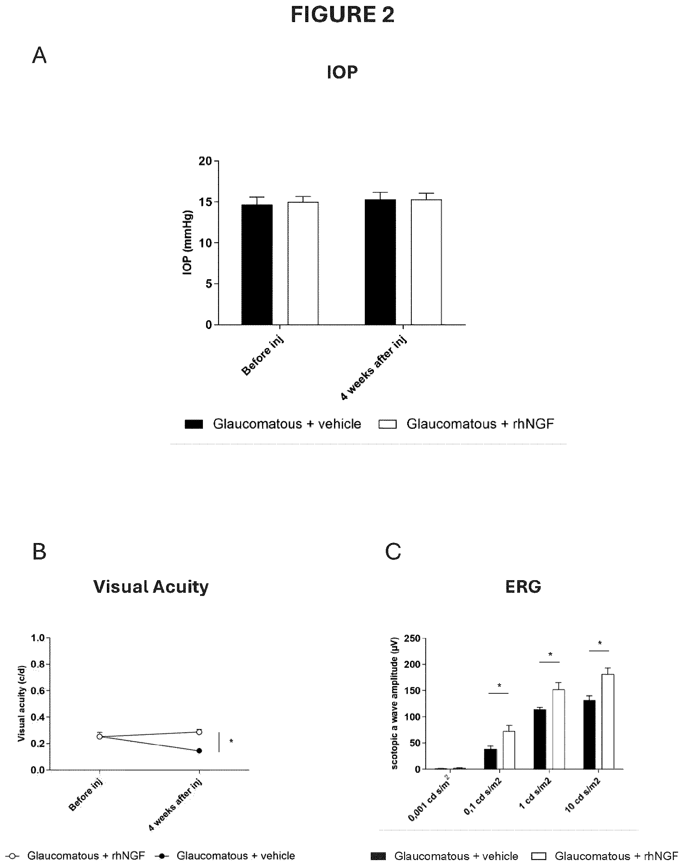

**Figure 2** shows the results of (A) intraocular pressure ("IOP", mmHg) and (B) visual acuity (c/d) analysis, before rhNGF or vehicle intravitreal injection ("Before inj."), and 4 weeks after rhNGF or vehicle intravitreal injection ("4 weeks after inj.") in 7-month-old animal groups, and the results of (C) scotopic a wave amplitude (µV) of ERG (at 0.001 cd·s/m², 0.1 cd·s/m², 1 cd·s/m² and 10 cd·s/m²) analysis of vehicle-treated glaucoma ("Glaucomatous + vehicle") and rhNGF-treated glaucoma ("Glaucomatous + rhNGF") 7-month-old animal groups, according to Example 1. Two-tailed Student's t-test. *p<0.05 vs. vehicle-treated glaucoma ("Glaucomatous + vehicle").

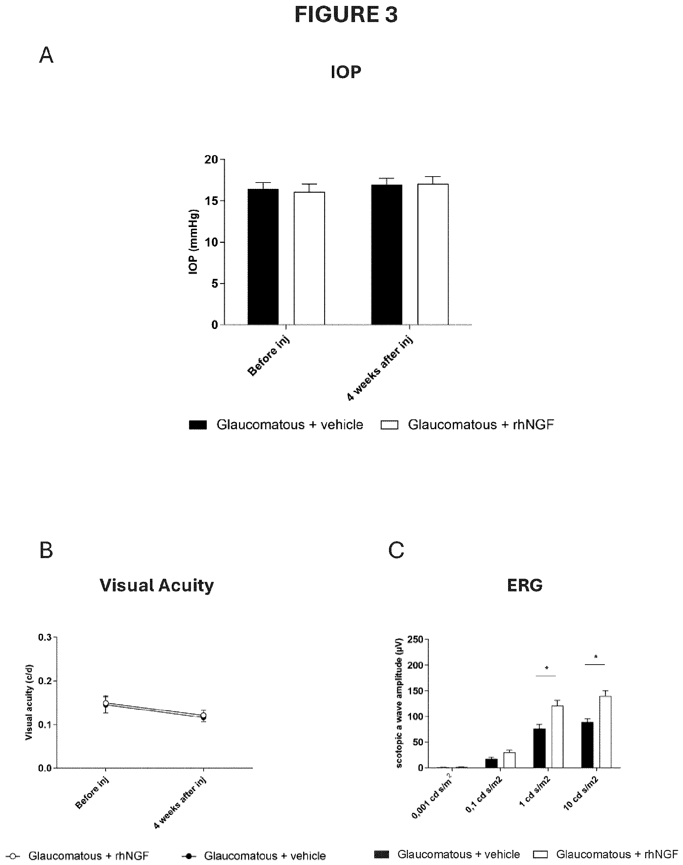

**Figure** 3 shows the results of (A) intraocular pressure ("IOP", mmHg) and (B) visual acuity (c/d) analysis, before rhNGF or vehicle intravitreal injection ("Before inj."), and 4 weeks after rhNGF or vehicle intravitreal injection ("4 weeks after inj.") in 10-month-old animal groups, and the results of (C) scotopic a wave amplitude (µV) of ERG (at 0.001 cd·s/m², 0.1 cd·s/m², 1 cd·s/m² and 10 cd·s/m²) analysis of vehicle-treated glaucoma ("Glaucomatous + vehicle") and rhNGF-treated glaucoma ("Glaucomatous + rhNGF") 10-month-old animal groups, according to Example 1. Two-tailed Student's t-test. *p<0.05 vs. vehicle-treated glaucoma ("Glaucomatous + vehicle").

### DETAILED DESCRIPTION OF THE INVENTION

A first object of the invention is nerve growth factor (NGF) for use in improving visual function in a subject suffering from glaucoma, wherein said NGF is administered intravitreally to said subject.

Preferably, said subject is a human subject.

Preferably, the improvement in a subject's visual function, according to the present invention, is in one or both eyes.

By "improving visual function" according to the present invention, it is meant improving visual function of one or both eyes of the subject compared to the visual function baseline of the same eye(s) of the subject, wherein by visual function baseline it is meant visual function prior to the start of the intravitreal administration of NGF.

In particular, the subject's visual function may be established by evaluating one or more of the subject's visual field, visual acuity, ability to perform life tasks, retinal sensitivity, dynamic pupillary response, nystagmus, cortical visual function, color vision or dark adaptation.

According to a preferred aspect of the present invention, said visual function may be established by evaluating visual acuity, visual field index (VFI), mean deviation (MD) or pattern standard deviation (PSD), ganglion cell layer and retinal nerve fiber layer (RNFL) thickness, contrast sensitivity or pattern ERG.

Accordingly, in said preferred aspect of the present invention, by "improving visual function" according to the present invention it is meant improving visual acuity, visual field index (VFI), mean deviation (MD) or pattern standard deviation (PSD), ganglion cell layer and retinal nerve fiber layer (RNFL) thickness, contrast sensitivity or pattern ERG baseline of the same eye(s) of the subject, wherein by visual acuity, visual field index (VFI), mean deviation (MD) or pattern standard deviation (PSD), ganglion cell layer and retinal nerve fiber layer (RNFL) thickness, contrast sensitivity or pattern ERG baseline it is meant visual acuity, visual field index (VFI), mean deviation (MD) or pattern standard deviation (PSD), ganglion cell layer and retinal nerve fiber layer (RNFL) thickness, contrast sensitivity or pattern ERG prior to the start of the first intravitreal administration of NGF.

Preferably, improving of visual function is determined by functional assessments, for example as mean, median, and distribution of change from baseline in best corrected visual acuity (BCVA) (measured in Early Treatment Diabetic Retinopathy Study [ETDRS] charts), a change from baseline of pointwise linear regression, as assessed by Humphrey Visual Field (HVF) analysis using the SAP 24-2 SITA standard protocol; a change from baseline of visual field index (VFI), mean deviation (MD) and pattern standard deviation (PSD), assessed by Humphrey Visual Field Analyzer (HVFA) using the SAP 24-2 -SITA standard; changes from baseline in photopic negative response (PhNR) on the electroretinography (ERG); a change from baseline in contrast sensitivity, or from baseline of pattern ERG, or baseline in glaucoma quality of life questionnaire (GQL15).

Said known methods may be also used for determining or assessing the function of retina and/or optic nerve.

Preferably, the improvement of visual function is determined between 8 weeks and 12 weeks after the intravitreal administration of NGF.

Preferably, said glaucoma is open-angle glaucoma, angle-closure glaucoma or congenital glaucoma. More preferably, said glaucoma is open-angle glaucoma.

Preferably, said glaucoma is in the early, intermediate or advanced stage, more preferably in the intermediate or advanced stage.

According to the present invention:
By "glaucoma in an early stage", it is meant a glaucoma wherein the mean deviation in the Humphrey 24-2 visual field (VF) test is better than -6dB is measured.
By "glaucoma in intermediate stage", it is meant a glaucoma wherein the mean deviation in the Humphrey 24-2 visual field (VF) test is between -6.01 and -12 dB.
By "glaucoma in advanced stage", it is meant a glaucoma wherein the mean deviation in the Humphrey 24-2 visual field (VF) test is between -12.01 and -20 dB.
By "glaucoma in late stage", it is meant a glaucoma wherein the mean deviation in the Humphrey 24-2 visual field (VF) test worse than -20.01 dB.

Preferably said NGF is human NGF.

Preferably, said human NGF has the amino acid sequence of SEQ. ID NO.1 below.
SEQ. ID NO.1:

SSSHPIFHRGEFSVCDSVSVWVGDKTTATDIKGKEVMVLGEVNINNSVFKQYFFETKCRD
PNPVDSGCRGIDSKHWNSYCTTTHTFVKALTMDGKQAAWRFIRIDTACVCVLSRKAVR

Alternatively, said human NGF has the amino acid sequence of SEQ ID NO. 2 below:
SEQ ID NO. 2:

SSSHPIFHRGEFSVCDSVSVWVGDKTTATDIKGKEVMVLGEVNINNSVFKQYFFETKCRD
PNPVDSGCRGIDSKHWNSYCTTTHTFVKALTMDGKQAAWRFIRIDTACVCVLSRKAVRR
A

Alternatively, said human NGF is a mixture of NGFs having sequences of SEQ ID NO. 1 and SEQ ID NO. 2.

The human NGF of SEQ ID NO. 2 has an amino acid sequence that only differs from the NGF of SEQ ID NO. 1 for the presence of two additional amino acids at the C-terminus. Both forms of NGF are found in human cells and therefore are considered wild type human NGF.

Therefore, when referring to "human NGF" or "wild-type human NGF", in the present application, it is meant a human NGF of SEQ ID NO. 1 or SEQ ID NO. 2 or mixtures thereof.

Preferably, said NGF is produced by recombinant DNA technology, preferably it is a human recombinant NGF (rhNGF). Methods of producing rhNGF are known to the person skilled in the art, for example those described in WO0022119A1 and WO2013092776A1.

Preferably, the NGF for use according to the invention is administered to said subject by intravitreal injection in an amount/eye per each administration between 0.1 µg and 20 µg, more preferably between 0.5 µg and 10 µg, even more preferably between 1 µg and 5 µg, most preferably of 1,1.5, 2 or 3 µg.

Preferably, the NGF for use according to the invention is administered to said subject by intravitreal injection in a volume per each administration comprised between 15 µl and 35 µl, more preferably between 20 µl and 30 µl, most preferably is 25 µl.

The administration schedule may vary depending on the patient and it is selected by the skilled person based on the characteristics of the subject to be treated, the severity of the disease and the tests carried out during the treatment to monitor response and progression of disease.

Preferably, the NGF for use according to the invention is administered to said subject more than once according to clinical evaluation of the patient. Preferably, the frequency of administration is not more often than once every four months, more preferably not more often than once every six months, even more preferably not more often than once a year.

A further object of the present invention is a pharmaceutical composition comprising said nerve growth factor (NGF), as disclosed above, and at least one pharmaceutically acceptable excipient, for use in improving visual function, as described above, in a subject suffering from glaucoma, as described above, said pharmaceutical composition being administered intravitreally to said subject.

By "pharmaceutically acceptable excipient" as used herein, it is meant an excipient which is suitable for administration to the eye of a subject.

Preferably, said pharmaceutically acceptable excipient is selected from solvents, thickening agents, mucoadhesive agents, buffers, antioxidants and preservatives.

Said at least one pharmaceutically acceptable excipient may be selected from the group comprising: calcium chloride; carboxymethylcellulose sodium; DL-lactide and glycolide (50:50) copolymer 12000 acid; hyaluronate sodium; hydrochloric acid; magnesium chloride; magnesium stearate, microcrystalline cellulose; polyvinyl alcohol; potassium chloride; sodium acetate; sodium bicarbonate; sodium chloride; sodium hydroxide; sodium phosphate, dibasic, heptahydrate; sodium phosphate, monobasic, monohydrate; trisodium citrate dihydrate; citric acid monohydrate; sodium metabisulfite; sodium phosphate, dibasic, anhydrous; sodium phosphate, monobasic, anhydrous; sucrose; histidine; acetic acid; L-methionine; and trehalose.

Preferably, said pharmaceutical composition is a pharmaceutical liquid composition.

Said pharmaceutical liquid composition may be in the form of a solution or suspension, preferably it is a solution.

Preferably, said pharmaceutical liquid composition contains a physiological saline solution (0.9% sodium chloride (NaCl)), as a major vehicle.

More preferably, said pharmaceutical liquid composition comprises, even more preferably consists of, NGF, preferably rhNGF, and physiological saline solution (0.9% sodium chloride (NaCl).

Preferably, the pH of said pharmaceutical liquid composition is between 4.5 and 8.0, preferably around 7.

Preferably, the concentration of NGF in said pharmaceutical liquid composition is between 20 µg/ml and 180 µg/ml, more preferably between 40 µg/µl and 140 µg/µl, even more preferably selected from 60 µg/ml and 120 µg/ml.

Preferably, the volume of said pharmaceutical liquid composition is comprised between 15 µl and 35 µl, more preferably between 20 µl and 30 µl, most preferably is 25 µl.

The pharmaceutical liquid composition according to the invention may be suitably formulated using appropriate methods known in the art or by the method disclosed in Remington's Pharmaceutical Science (recent edition), Mack Publishing Company, Easton Pa.

In a further aspect, the present invention relates to a method for improving visual function, as described above, in a subject suffering from glaucoma, as above described, comprising administering to the subject NGF, as described above, in a therapeutically effective amount by intravitreal injection.

The invention will be further described in the following examples, which do not limit the scope of the invention defined in the claims.

### EXPERIMENTAL SECTION

### Example 1

The pharmaceutical efficacy in treating glaucoma of intravitreal injections of rhNGF (SEQ ID No:1) on eyes of DBA/2J (Strain #:000671, obtained from the European distributor of Jackson Laboratories Mice (Charles Rivers Laboratories, Wilmington, MA, USA) mice was tested, according to the below.

Mice were housed in macrolon cages with filter hoods, in a room where the air is continuously filtered, thereby avoiding contamination. During experiments, paired animals were caged at a constant temperature with a day/night cycle of 12/12 hours.

Animals received water (control tap water) and nutrition *ad libitum.* Animal protocol is approved by the Italian ministry of health (approval code CE5C5.34) and performed by Invivex SAS with animal protocol approved by the Animal Studies Committee of Languedoc Roussillon (Reference Number: D3417223, APAFIS#23920-2020020320279696v3). Animal health will be examined every day to ensure that only animals in good health enter the testing procedures and follow up the study.

### Animal models and treatments:

DBA/2J mice are widely used as an *in vivo* model for the study of glaucoma, as they spontaneously develop glaucoma in a progressive and age-dependent manner (Amato et al, Cells 2023 (12), 1272).

Accordingly, to analyze the therapeutic effect of rhNGF at various stages of development of the pathology, three groups of animals were treated: a mice group of 4 months of age; a mice group of 7 months of age, and a mice group of 10 months of age.

In particular, the glaucoma condition of said 4-5 months mice group corresponds to the "early stage" glaucoma of human subjects as defined in the present invention; the glaucoma condition of said 6-9 months mice group corresponds to the "intermediate stage" and "advanced stage" glaucoma of human subjects as defined in the present invention; and the glaucoma condition of said 10 months mice group corresponds to the "late stage" glaucoma of human subjects as defined in the present invention.

Said groups were treated by intravitreal administration of a rhNGF solution (consisting of 1 µg/µl rhNGF, 0.9% saline (sodium chloride) solution), or a vehicle solution (consisting of 0.9% saline (sodium chloride) solution), according to the following:
- *Early stage of glaucoma:*
   -- a group of 10, 4-month-old DBA/2J mice received a single intravitreal injection of the rhNGF solution ("Glaucomatous + rhNGF");
   -- a group of 10, 4-month-old DBA/2J mice received a single intravitreal injection of the vehicle solution ("Glaucomatous + vehicle").
- *Intermediate and advanced stage of glaucoma:*
   -- a group of 10, 7-month-old DBA/2J mice received a single intravitreal injection of the rhNGF solution ("Glaucomatous + rhNGF");
   -- a group of 10, 7-month-old DBA/2J mice received a single intravitreal injection of a vehicle solution ("Glaucomatous + vehicle").
- *Late stage of glaucoma:*
   -- a group of n=10, 10-month-old DBA/2J mice received a single intravitreal injection of a solution of said rhNGF in *both eyes* ("Glaucomatous + rhNGF");
   -- a group of n=10, 10-month-old DBA/2J mice received a single intravitreal injection of a vehicle solution in *both eyes* ("Glaucomatous + vehicle");

All said DBA/2J mice were diagnosed with glaucoma, and specifically selected for an IOP ≥ 11 mmHg.

In order to perform intravitreal administrations, anesthesia was performed with an intraperitoneal injection of ketamine [50 mg/kg] /xylazine [10 mg/kg] mixture; then superior nasal region of the eye of the animal was exposed and a glass needle was inserted at a 45° angle through the sclera into the vitreous body. A small amount of vitreous was rid through the puncture hole from the posterior chamber by the eyeball mild pressure. Then, 1 µl of the above rhNGF solution or vehicle solution was injected into the posterior chamber and the needle was kept in place for few seconds.

In particular, a single injection of rhNGF/Vehicle (1 µg/eye, 1 µl per eye) solution has been injected only into the left eye of each mouse.

After intravitreal injection, a drop of ophthalmic Tobrex solution was applied on the mouse eye to prevent infection.

### - Intraocular pressure (IOP), visual acuity and electroretinography (ERG) analysis.

The following analysis of intraocular pressure (IOP), visual acuity and electroretinography (ERG) were carried out the day before the administration of said rhNGF solution or vehicle solution ("Before compound administration"), according to the groups above, and 4 weeks after intravitreal administration of said rhNGF solution or vehicle solution ("4 weeks after compound administration").

### Intraocular pressure (IOP) measure

Tonolab system (Icare LAB tonometer, TonoLab, Vantaa, Finland) was used to quantitatively assess the intraocular pressure in rodents at indicated time points. The animals were immobilized with the left hand. Then, the tonometer was brought near the mouse eye with the groove in a horizontal position. The distance between the tip of the probe and the mouse cornea was kept at 1 to 3 millimeters. Then, the tip of the probe was advanced until contact with the central cornea, and the measuring button was pressed six times. Once the six correct single measurements were done, the IOP was shown on display. Final IOP reading was an average of 4 mean single readings. IOP measurements were performed before intraocular injection (baseline) and at endpoint.

### Visual acuity measure

The visual acuity of the above tested animals was determined by the following optometry test. The animals were individually placed on a platform in the center of a cylinder. Right eye was closed using histoacryl. Animals were adapted to the environment for 5 min. The mouse number and condition were selected on the software. The step size of the stimulus was selected manually. The stimulation conditions were: 0.05 c/d sinusoidal for the spatial frequency, speed 12 d/s and temporal frequency 0.6 Hz. The reflexive head and neck movements were noted for each animal. The improvement of the spatial frequency measured in cycles/degree (c/d) was measured. Each animal performed 3 trials at 5 minutes intervals. For each day, values from the 3 trials were averaged for each animal and then averaged for each treated group.

### Electroretinography (ERG)

Mice were anesthetized using ketamine [50 mg/kg] /xylazine [10mg/kg] mixture individually. Eye was treated with 1% atropine sulphate, 2.5% phenylephrine hydrochloride and 0.5% proparacaine hydrochloride, allowing drops to sit on the eyes for ~2 min before wicking with a cotton swab and applying the next drop. Then, the mouse was positioned on the heated platform. Right eye was closed using histoacryl. The ground needle electrode was placed in the base of the tail, reference needle electrode subdermally between the eyes, a drop of 2.5% hypromellose was applied, and the contact lens electrodes was placed onto the cornea. The scotopic a-wave (dark-adapted) ERG measures were performed at 0.001 cd s/m2; 0.1cd s/m2; 1 cd s/m2; and 10 cd s/m2 with pulse frequency: 0.2 Hz, sample frequency: 1.000 Hz, trial pre-trigger time: 20 msec and trial post-trigger time: 250 msec (Ganzfeld ColorDome, Diagnosys, catalogue number: D125). The amplitude values of left eyes were averaged automatically for each animal and then averaged for each group. At the end of the measure, electrodes were removed from the mouse and the animal was placed in a clean cage on top of a heat pad until recovering. The day before ERG analysis, all mice were placed in a light-proof dark room allowing them to dark-adapt overnight.

### Statistical analysis

All data are presented as mean ± standard deviation (SD) unless stated otherwise. Statistical significance was determined by two-tailed Student's t-test. p-values < 0.05 were considered statistically significant. Analyses used GraphPad Prism version 9.0, GraphPad Software (San Diego, CA).

### - Results

### -- Early stage of glaucoma

The above-reported group of 4-month-old mice allowed to evaluate the effect of said intravitreal injection of rhNGF on the initial molecular events of the onset of glaucomatous pathology.

No effect of rhNGF injection on IOP was observed in the rhNGF treated group ("Glaucomatous + rhNGF"), compared to the IOP vehicle-treated group ("Glaucomatous + vehicle"), both before the intravitreal injection and 4 weeks after the intravitreal injection as shown in Figure 1A. A slight increase in IOP was observed compared to prior of treatment for both rhNGF treated and vehicle-treated groups.

The results of the visual acuity test are reported in Figure 1B.

The rhNGF-treated group ("Glaucomatous + rhNGF") showed a significantly recovery of visual acuity not observed in the vehicle-treated group ("Glaucomatous + vehicle").

The results of the ERG analysis are reported in Figure 1C.

The intravitreal administration of rhNGF demonstrated an unexpected recovery in retinal function in rhNGF-treated group ("Glaucomatous + rhNGF"), compared to vehicle-treated group ("Glaucomatous + vehicle").

The results obtained demonstrate that rhNGF administered intravitreally improves visual function in early stage of glaucoma compared to the vehicle-treated glaucoma group.

### -- Intermediateladvanced stage of glaucoma:

The above-reported group of 7-month-old mice allowed to clarify how intravitreal rhNGF influences the molecular and cellular events of an already partially damaged glaucomatous retina.

Surprisingly, even in this glaucoma animal model, no effect of rhNGF injection on IOP was observed in the rhNGF treated group ("Glaucomatous + rhNGF"), compared to the IOP vehicle-treated group ("Glaucomatous + vehicle"), both before the intravitreal injection and 4 weeks after the intravitreal injection as shown in Figure 2A. Furthermore, no significant increase in IOP was observed compared to prior of treatment for both rhNGF-treated and vehicle-treated groups.

The results of the visual acuity test are reported in Figure 2B.

As shown in above-reported results of the treatment of the early stage of glaucoma, the rhNGF-treated group ("Glaucomatous + rhNGF") showed a significantly recovery of visual acuity not observed in the vehicle-treated group ("Glaucomatous + vehicle").

The results of the ERG analysis are reported in Figure 2C.

The intravitreal administration of rhNGF demonstrated an unexpected recovery in retinal function in rhNGF-treated group ("Glaucomatous + rhNGF"), compared to vehicle-treated group ("Glaucomatous + vehicle"), even at 0.1 cd s/m².

As shown for the early stage of glaucoma, rhNGF administered intravitreally improves visual function also in intermediate/advanced stage of glaucoma compared to the vehicle-treated glaucoma group.

### -- Late stage of glaucoma:

The above-reported group of 10-month-old mice allowed to evaluate whether intravitreal treatment with rhNGF is effective in the late phase of glaucomatous pathology.

The rhNGF-treated group ("Glaucomatous + rhNGF") and the vehicle-treated group ("Glaucomatous + vehicle") showed similar IOP at both before rhNGF or vehicle intravitreal administration, and 4 weeks after rhNGF or vehicle intravitreal administration, suggesting the achievement of a plateau of the internal eye pressure, at this stage of glaucoma, as shown in Figure 3A.

The results of visual acuity are reported in Figure 3B.

A reduction trend in visual acuity in both "Glaucomatous + rhNGF" and "Glaucomatous + vehicle" groups, with no statistical difference between these groups, is shown, suggesting a lack of efficacy of rhNGF in improving the visual acuity in the tested late glaucomatous stage animal model.

However, an improvement of the visual function is obtained also in said late glaucomatous stage animal model, as shown by the results of the ERG analysis, reported in Figure 3C, which clearly show a surprising enhancement in retinal function in rhNGF-treated group ("Glaucomatous + rhNGF"), compared to vehicle-treated group ("Glaucomatous + vehicle"), at both at 1 cd s/m² and at 10 cd s/m².

## Claims

1. Nerve growth factor (NGF) for use in improving visual function in a subject suffering from glaucoma, wherein said NGF is administered intravitreally to said subject.

2. NGF for use as claimed in claim 1, wherein the amount/eye per each administration between 0.1 µg and 20 µg, preferably between 0.5 µg and 10 µg, more preferably between 1 µg and 5 µg, even more preferably of 1, 1.5, 2 or 3 µg.

3. NGF for use as claimed in claim 1 or 2, said NGF is human NGF, more preferably it is recombinant human NGF.

4. NGF for use as claimed in claims 1 to 3, wherein said human NGF consists of the amino acid sequence of SEQ ID No. 1 or SEQ ID No. 2 or mixtures thereof.

5. A pharmaceutical composition comprising nerve growth factor (NGF) and at least one pharmaceutically acceptable excipient, for use in improving visual function in a subject suffering from glaucoma, said pharmaceutical composition being administered intravitreally to said subject.

6. A pharmaceutical composition for use as claimed in claim 5, wherein said NGF is present in the composition at a concentration between 20 µg/ml and 180 µg/ml, preferably between 40 µg/µl and 140 µg/µl, more preferably selected from 60 µg/ml and 120 µg/ml.

7. A pharmaceutical composition for use as claimed in claim 5 or 6, wherein said NGF is human NGF, preferably it is recombinant human NGF.

8. A pharmaceutical composition for use as claimed in claims 5 to 7, wherein said human NGF consists of the amino acid sequence of SEQ ID No. 1 or SEQ ID No. 2 or mixtures thereof.

9. NGF for use as claimed in claims 1 to 4, or said pharmaceutical composition for use as claimed in claims 5 to 8, wherein said glaucoma is open-angle glaucoma, angle-closure glaucoma or congenital glaucoma, preferably open-angle glaucoma.

10. NGF for use as claimed in claims 1 to 4, or claim 9, or said pharmaceutical composition for use as claimed in claims 5 to 8, or claim 9, wherein said glaucoma is in the early, intermediate or advanced stage, preferably in the intermediate or advanced stage.
